# FASCICULE DE BREVET EUROPEEN

(11) **EP 2 167 142 B1**
(45) Date de publication et mention de la délivrance du brevet: **10.11.2010**
(21) Numéro de dépôt: 08787904.5
(22) Date de dépôt: 03.04.2008
(51) Int. Cl.: A61L 2/24, A61L 2/26, A61B 19/00, G06F 19/00

(54) **DISPOSITIF DE CONDITIONNEMENT D'UN APPAREIL MEDICAL DE TYPE SONDE**
VORRICHTUNG ZUR KONFIGURATION EINER SONDENARTIGEN MEDIZINISCHEN VORRICHTUNG
DEVICE FOR CONDITIONING A MEDICAL APPARATUS OF THE PROBE TYPE

(30) Priorité: 05.04.2007 FR 0754300
(43) Date de publication de la demande: 31.03.2010
(73) Titulaire: Germitec, 75008 Paris (FR)
(72) Inventeur: DESHAYS, Clément, F-07120 Ruoms (FR)
(86) Numéro de dépôt international: PCT/FR2008/000465
(87) Numéro de publication internationale: WO 2009/004127

(56) Documents cités:
- WO-A-01/80908
- WO-A-2007/016101
- FR-A- 2 890 864
- DATABASE WPI Week 200706 Thomson Scientific, London, GB; AN 2007-056585 XP002432691 & WO 2006/115177 A1 (OLYMPUS MEDICAL SYSTEMS CORP) 2 novembre 2006 (2006-11-02)

## Description

La présente invention concerne un dispositif de conditionnement d'un appareil médical du type sonde ou similaire, ledit dispositif formant une enceinte interne destinée à recevoir au moins une partie de l'appareil médical, ladite enceinte pouvant être fermée hermétiquement, ledit dispositif comprenant des moyens de verrouillage de la fermeture de l'enceinte. L'invention concerne également un système de suivi de l'appareil médical du type sonde comprenant des moyens d'identification dudit appareil portés par l'appareil médical, au moins un système d'acquisition et de traitement d'informations comprenant des moyens d'acquisition des informations d'identification dudit appareil, des moyens d'acquisition d'informations d'utilisation relatives à l'utilisation dudit appareil et d'informations de désinfection relatives à la désinfection dudit appareil, des moyens d'horodatage et des moyens d'association des informations d'identification, d'utilisation, de désinfection dudit appareil et d'horodatage.

Les documents FR-A-2890864 et WO-A-01/80908 divulguent des dispositifs de stérilisation d'appareils médicaux, comprenant une enceinte de stérilisation destinée à recevoir les appareils et des moyens électroniques de verrouillage empêchant l'ouverture de ladite enceinte pendant les cycles de stérilisation.

Dans le domaine médical, l'hygiène des appareils médicaux employés est primordiale afin d'éviter tout risque d'infection d'un patient à cause d'un appareil pas ou mal désinfecté. C'est pourquoi il est indispensable d'assurer une traçabilité de la désinfection afin d'être sûr qu'un appareil a bien été désinfecté avant son utilisation.

Or, actuellement, cette traçabilité est assurée par des opérations manuelles du personnel médical. Ainsi, une fiche accompagne la sonde médicale sur laquelle le personnel médical doit inscrire les opérations de désinfection qui ont été effectuées, ainsi que la façon dont la sonde est utilisée. Dans certains cas, la traçabilité est partiellement automatisée et les informations relatives à une sonde sont regroupées dans une base de données. Cependant, dans ce dernier cas, le personnel médical doit tout de même entrer lui-même les informations relatives à la désinfection et à l'utilisation de la sonde dans la base de données. Par conséquent, la traçabilité est assurée au travers de beaucoup d'interventions humaines qui peuvent être source d'erreur et/ou de falsifications. Il est donc impossible de savoir de façon absolument certaine si une sonde peut être utilisée de façon légitime du point de vue hygiénique et de la lutte contre les infections nosocomiales.

La sonde médicale peut être protégée entre les phases de désinfection et d'utilisation de l'appareil au moyen d'un dispositif de conditionnement formant une enceinte étanche recevant la partie active de la sonde. Cependant, c'est un opérateur qui décide quand introduire la sonde dans l'enceinte étanche et quand l'en sortir afin de permettre son utilisation. Dans une telle réalisation, il n'y a pas de vérification, autre qu'une vérification faisant intervenir une personne, que la sonde a bel et bien été désinfectée avant son introduction dans le dispositif de conditionnement et que cette sonde est apte à être utilisée avant de sortir la sonde du dispositif de conditionnement.

L'invention vise à pallier ces inconvénients en proposant un dispositif de conditionnement et un système de suivi évitant toute intervention humaine pour garantir que la sonde est utilisable légitimement du point de vue hygiénique.

A cet effet, l'invention concerne un dispositif de conditionnement du type précité, dans lequel ledit dispositif est **caractérisé en ce qu**'il comprend des moyens de réception d'informations d'identification en provenance de l'appareil médical des moyens de réception d'informations de désinfection et d'utilisation en provenance d'au moins un système de traitement d'informations, lesdits moyens de verrouillage étant agencés pour se déclencher si les moyens de réception reçoivent une information selon laquelle l'appareil médical identifié qu'il doit recevoir a été désinfecté et pour empêcher le verrouillage si les moyens de réception reçoivent une information selon laquelle l'appareil médical n'a pas été désinfecté.

Ainsi, le verrouillage de l'enceinte de conditionnement ne se produit que si la sonde a été préalablement désinfectée sans que l'opérateur n'ait à s'en assurer par lui-même, l'information se transmettant de façon automatique. Si le dispositif empêche le verrouillage de la fermeture, l'opérateur sait que la sonde doit être désinfectée avant d'être amenée vers son point d'utilisation.

Selon d'autres caractéristiques du dispositif de conditionnement :
- le dispositif de conditionnement comprend des moyens d'émission d'informations relatives à l'appareil médical qu'elle contient vers le système de traitement d'informations, lesdits moyens d'émission étant agencés pour émettre l'information d'identification de l'appareil médical reçu dans l'enceinte au système de traitement d'informations lorsque les moyens de verrouillage ont verrouillé la fermeture de l'enceinte ;
- les moyens de verrouillage sont agencés pour déverrouiller la fermeture de l'enceinte lorsque les moyens de réception reçoivent une information selon laquelle l'appareil médical va être utilisé du système de traitement d'informations ;
- les moyens d'émission sont agencés pour informer le système de traitement d'informations lorsque la fermeture de l'enceinte est déverrouillée et que l'appareil médical est sorti de l'enceinte ;
- les moyens d'émission envoient les informations relatives à l'identification et à la désinfection de l'appareil médical que le dispositif contient dans son enceinte au système de traitement des informations lorsque les moyens de verrouillage déverrouille la fermeture de l'enceinte ;
- le dispositif de conditionnement comprend des moyens d'horodatage, les moyens de verrouillage étant agencés pour déverrouiller la fermeture de l'enceinte lorsque l'appareil médical est présent dans l'enceinte depuis un temps prédéterminé ;
- les moyens d'émission et de réception d'informations sont des moyens fonctionnant à distance ; et
- les moyens d'émission et de réception d'informations comprennent des moyens de connexion filaires.

L'invention concerne également un système de suivi du type précité, dans lequel ledit système est **caractérisé en ce qu**'il comprend un dispositif de conditionnement tel que défini ci-dessus, le dispositif de conditionnement recevant l'appareil médical entre les phases de désinfection et d'utilisation de l'appareil, le dispositif de conditionnement, l'appareil médical, le système d'acquisition et de traitement d'informations étant aptes à communiquer entre eux afin de se transmettre des informations relatives à l'identification de l'appareil médical, à sa désinfection et à son utilisation.

Un tel système de suivi permet de déterminer si une sonde a été désinfectée et peut être utilisée légitimement du point de vue hygiénique sans intervention humaine, le système est en outre apte à empêcher cette utilisation en cas de non respect de la chaîne hygiénique. Ainsi, le risque d'erreur de manipulation est supprimé.

Selon d'autres caractéristiques du système de suivi :
- les appareils médicaux, du type enceinte de désinfection, enceinte de stockage, appareil d'imagerie, avec lesquels l'appareil médical de type sonde doit fonctionner comprennent chacun des moyens d'acquisition des informations d'identification, des moyens d'acquisition d'informations de désinfection et d'informations d'utilisation et des moyens d'horodatage ;
- les appareils médicaux, du type enceinte de désinfection, enceinte de stockage, appareil d'imagerie, avec lesquels l'appareil médical de type sonde doit fonctionner comprennent chacun un système d'acquisition et de traitement d'informations ; et
- les moyens d'acquisition des informations d'identification, les moyens d'acquisition de désinfection et d'informations d'utilisation, les moyens d'horodatage sont regroupé au sein d'un appareil indépendant et portable apte à communiquer avec un système de traitement d'informations.

D'autres caractéristiques et avantages de l'invention apparaîtront au cours de la description qui suit, faite à titre d'exemple et en référence aux dessins annexés sur lesquels :
- la Fig. 1 est une représentation schématique en perspective d'un dispositif de conditionnement selon l'invention et d'une sonde destinée à être introduite dans le dispositif de conditionnement.
- la Fig. 2 est un est un diagramme représentant les différentes étapes de fonctionnement du système de suivi selon l'invention.

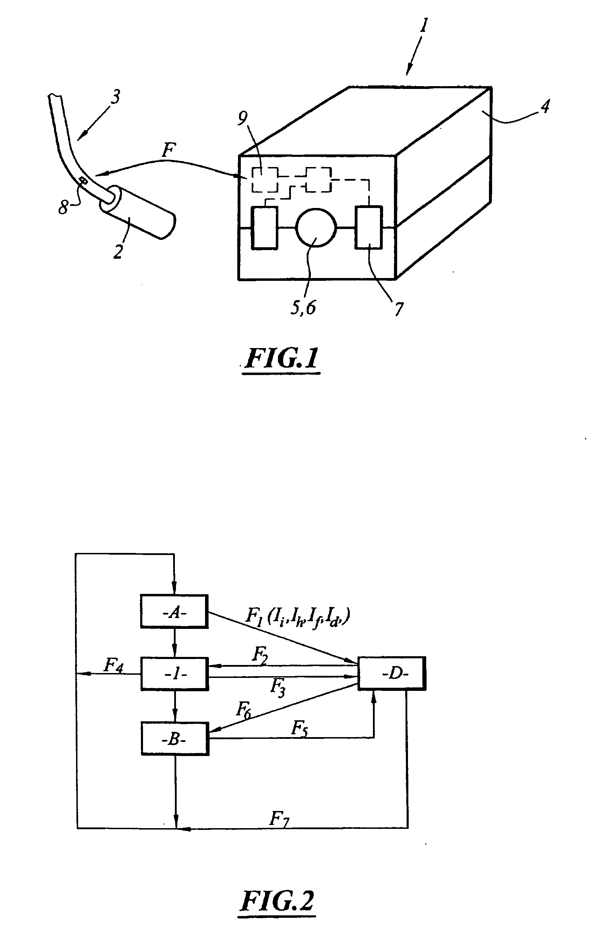

La Fig. 1 montre un dispositif de conditionnement mobile 1 destiné à recevoir une partie 2 d'un appareil médical 3 du type sonde. La partie 2 reçue par le dispositif de conditionnement 1 est typiquement la partie active de la sonde destinée à être en contact avec un patient et qui doit donc être désinfectée afin d'éviter tout risque d'infection.

Le dispositif de conditionnement mobile 1 comprend un boîtier 4 formant une enceinte interne 5 à l'intérieur de laquelle la partie 2 de la sonde 3 est destinée à être disposée en passant par une ouverture 6.

L'enceinte 5 est agencée pour être hermétiquement fermée lorsque la partie 2 de la sonde 3 est introduite à l'intérieur. A cet effet, l'ouverture 6 est munie de moyens d'étanchéité (non représentés) du type joint, diaphragme ou similaire.

Le boîtier 4 peut être ouvert en vue de l'introduction de la partie 2 de la sonde puis fermé hermétiquement afin de sceller l'enceinte 5.

Le dispositif comprend des moyens de verrouillage 7 de la fermeture de l'enceinte 5 permettant d'empêcher l'ouverture de l'enceinte 5 si les moyens de verrouillage sont activés. Selon une réalisation, le dispositif de conditionnement 1 est autoclavable.

Les moyens de verrouillage sont commandés de façon électronique pour se déclencher et verrouiller la fermeture de l'enceinte 5 en fonction d'informations reçues par le dispositif de conditionnement 1, comme cela va être décrit ultérieurement.

Le dispositif de conditionnement 1 appartient à un système de suivi de l'utilisation d'une sonde 3 permettant de vérifier qu'une sonde 3 peut être utilisée légitimement du point de vue hygiénique, c'est-à-dire qu'elle a été désinfectée de façon adéquate et suffisamment récemment avant toute utilisation.

Le système de suivi comprend des moyens d'identification 8 de la sonde 3 portés par la sonde 3. Ces moyens sont par exemple une puce RFID fixée à demeure sur la sonde 3 et contenant des informations d'identification de la sonde. Ces moyens d'identification peuvent être lus à distance par des moyens d'acquisition 9 des informations d'identification de la sonde. Le dispositif de conditionnement 1 comprend de tels moyens d'acquisition 9, par exemple un lecteur RFID, qui peuvent lire les informations d'identification de la sonde 3, comme représenté par la flèche F de la Fig. 1. Ainsi, le dispositif de conditionnement 1 « reconnaît » la sonde 3 qu'il contient.

Le système de suivi comprend en outre au moins un système d'acquisition et de traitement d'informations D comprenant des moyens d'acquisition des informations d'identification de la sonde 3, des moyens d'acquisition d'informations d'utilisation relatives à l'utilisation de la sonde 3 et d'informations de désinfection relatives à la désinfection de la sonde 3, des moyens d'horodatage et des moyens d'association des informations d'identification, d'utilisation, de désinfection de la sonde 3 et d'horodatage. L'ensemble de ces moyens, rattachés à une base de données, permet d'assurer le suivi de la désinfection et de l'utilisation de la sonde.

Ainsi, au cours d'une étape A représentée sur la figure 2, la sonde est désinfectée. Préalablement à cette désinfection, la sonde est identifiée et la date de la désinfection est enregistrée, ainsi que l'heure de début de la désinfection. A la fin de la désinfection, l'heure de fin de la désinfection est enregistrée. Les informations d'identification Iᵢ, les informations de date et de début d'examen Iₕ et les informations de fin de désinfection I_{f} sont transmises à l'unité centrale de traitement des données D, comme représenté par la flèche F₁ de la figure 1. Un enregistrement de l'identité de l'opérateur effectuant la désinfection est également effectué.

Les moyens d'acquisition des informations d'identification, ainsi que les moyens d'horodatage peuvent être associés à une enceinte de désinfection ou faire partie d'un appareil indépendant et portable disposé dans la salle de désinfection.

Selon un mode de réalisation, on prévoit que l'enceinte de désinfection comporte des moyens d'identification de sorte que l'identité de l'enceinte utilisée pour désinfecter la sonde soit transmise à l'unité de traitement des données D. Selon un mode de réalisation, l'enceinte de désinfection comprend son propre système d'acquisition et de traitement d'informations permettant de relever les caractéristiques de la désinfection de la sonde.

La désinfection est ensuite réalisée. Des moyens d'acquisition d'informations relatives à l'utilisation dudit appareil permettent d'enregistrer les caractéristiques de la désinfection. Ces moyens d'acquisition comprennent par exemple des moyens de mesure du degré de désinfection fourni par une enceinte de désinfection. Les informations enregistrées comprennent par exemple l'enregistrement du principe actif utilisé pour la désinfection, le temps d'exposition à ce principe actif de l'appareil, la température de désinfection et/ou le dosage du principe actif. Ces mesures peuvent être effectuées au moyen de capteurs ou de tout autre moyens connus. Ces informations de désinfection I_{d} sont transmises à l'unité de traitement des données D qui détermine un degré de désinfection à partir de ces informations. Par exemple, si le dosage du principe actif est mesuré, différents degrés de désinfection seront atteints lorsque le dosage mesuré dépassera des seuils prédéterminés de dosage. Si un rayonnement UV est utilisé pour la désinfection, le temps d'exposition de la sonde à ce rayonnement combiné à la mesure du rayonnement délivré dans l'enceinte permet de déterminer le degré de désinfection.

Ainsi, à l'issue de l'étape A, le système de suivi de l'utilisation d'une sonde connaît le type, le degré et le moment de la désinfection de la sonde dont l'utilisation est suivie.

Le dispositif de conditionnement 1 comprend des moyens de réception d'informations de désinfection permettant lui permettant d'enregistrer à partir le système de traitement d'informations D, le type, le degré et le moment de la désinfection de la sonde 3 dont il connaît l'identité, comme représenté par le flèche F₂ de la Fig. 2.

Après que la désinfection a été effectuée, l'opérateur place la partie active de la sonde 3 dans le dispositif de conditionnement 1. Le dispositif de conditionnement 1 récupère les informations d'identification Iᵢ de la sonde 3, ainsi que les informations relatives à sa désinfection I_{d}.

Si le dispositif de conditionnement reçoit des informations selon lesquelles la désinfection a bien été réalisée à un degré adapté, alors le verrouillage de la fermeture de l'enceinte 5 est commandée électroniquement et la partie active 2 de la sonde 3 est maintenue hermétiquement dans l'enceinte 5.

Le dispositif de conditionnement 1 est pourvu de moyens d'émission d'informations relatives à la sonde qu'elle contient vers le système de traitement d'informations D. Ces moyens sont agencés pour informer le système de traitement d'informations D lorsque les moyens de verrouillage 7 ont verrouillé la fermeture de l'enceinte, comme représenté par la flèche F₃ de la Fig. 2. Ainsi, le système de traitement d'informations D peut dresser une liste de sondes 3 et des dispositifs de conditionnement 1 avec lesquels ces sondes 3 sont utilisées. Lorsque le système de traitement d'informations D a reçu une information selon laquelle une sonde 3 est disposée dans un dispositif de conditionnement 1, le système de traitement D communique l'identité de cette sonde 3 aux autres dispositifs de conditionnement 1 de sorte que ceux-ci n'accepteront pas une sonde portant les mêmes informations d'identification. Une telle réalisation permet de détecter des erreurs d'identification de sondes si des doublons ont été crées par erreur ou frauduleusement.

Si le dispositif de conditionnement récupère des informations selon lesquelles la désinfection n'a pas été réalisée ou qu'elle a été réalisée depuis un temps trop important ou à un degré insuffisant, le verrouillage de la fermeture de l'enceinte n'est pas réalisé et le dispositif de conditionnement informe le système de traitement d'informations D qu'une désinfection doit de nouveau avoir lieu par l'intermédiaire de ses moyens d'émission, comme représenté par la flèche F₃ de la Fig. 2. La sonde 3 est alors renvoyée à l'étape de désinfection A, comme représentée par la flèche F₄ de la Fig. 2. En outre, le système de traitement des données D émet une information selon laquelle la sonde 3 non désinfectée n'est pas acceptable par un autre dispositif de conditionnement 1. Ainsi, si un opérateur essaye d'utiliser un autre dispositif de conditionnement 1 pour stocker la sonde 3, ce dispositif refusera de verrouiller la fermeture de l'enceinte 5 au moment où il obtiendra les informations d'identification Iᵢ de la sonde 3.

Le dispositif de conditionnement 1 peut servir d'enceinte de stockage de la sonde 3 en attente d'utilisation. Selon un autre mode de réalisation, la sonde peut être placée avec le dispositif de conditionnement 1 dans une enceinte de stockage avant son utilisation.

Le dispositif de conditionnement 1 comprend des moyens d'horodatage aptes à déterminer la durée de stockage d'une sonde 3 dans son enceinte 4. Ainsi, si cette durée dépasse un temps prédéterminé, le dispositif de conditionnement 1 commande le déverrouillage de la fermeture de l'enceinte 4 et informe le système de traitement d'informations D qu'une désinfection doit de nouveau avoir lieu. La sonde 3 est alors renvoyée à l'étape de désinfection A et le système de traitement des données D émet une information selon laquelle la sonde 3 non désinfectée n'est pas acceptable par un autre dispositif de conditionnement 1.

Au cours d'une étape B, la sonde 3 est utilisée. Préalablement à l'utilisation, le médecin traitant informe le système de traitement de données D de son identité et des conditions d'utilisation prochaine de la sonde 3. Les conditions d'utilisation de la sonde 3 regroupent le nom du patient, le type de traitement pour lequel la sonde 3 est utilisée et tout autre information utile à ce traitement. A cet effet, l'appareil d'imagerie avec lequel la sonde 3 doit être utilisée comprend des moyens d'enregistrement de l'identité du médecin, tel qu'un lecteur de carte d'identification, ainsi que des moyens d'entrée des informations relatives aux conditions d'utilisation de la sonde. Le système de traitement de données D vérifie que la sonde 3 qui doit être utilisée peut l'être légitimement, c'est-à-dire si elle a été désinfectée de façon adaptée et suffisamment récemment. Si c'est le cas, le système de traitement envoie une information de déverrouillage au dispositif de conditionnement 1 qui commande alors le déverrouillage de la fermeture de l'enceinte 4, comme représenté par la flèche F₆ de la Fig. 2. Le médecin peut alors utiliser la sonde. Selon une réalisation, les informations d'indentification Iᵢ de la sonde de désinfection I_{d} sont transmises à l'appareil d'imagerie par le système de traitement de données D ou par le dispositif de conditionnement 1 lorsque le déverrouillage de la fermeture de l'enceinte 5 a été commandé.

La sonde 3 est identifiée et la date de l'examen est enregistrée. Cette identification et la date sont transmises à l'unité de traitement des données D, comme représenté par la flèche F₅ de la figure 1. L'unité de traitement croise ces informations et un séquençage entre la désinfection et l'utilisation est réalisé. Ainsi pour une identité de sonde donnée, toutes les informations relatives aux opérations précédant l'utilisation sont connues et mises en ordre chronologique. On s'assure ainsi qu'une chaîne d'opération a bien été menée avant de permettre l'utilisation de la sonde. Les opérations de vérification de l'identité de la sonde et de la légitimité d'utilisation peuvent être réalisées par l'appareil d'imagerie avec lequel la sonde est utilisée ou par l'appareil indépendant et portable déjà utilisé au cours de la désinfection et du stockage. Dans un tel cas, la commande de déverrouillage peut être émise par l'appareil d'imagerie lui-même ou par l'appareil indépendant et portable. Si la légitimité de l'utilisation de la sonde n'est pas garantie, le dispositif de conditionnement refusera de se déverrouiller et la sonde sera renvoyée à l'étape de désinfection, comme mentionné plus haut. Si l'appareil indépendant est utilisé, c'est celui-ci qui comprend des moyens d'enregistrement de l'identité du médecin, tel qu'un lecteur de carte d'identification, ainsi que des moyens d'entrée des informations relatives aux conditions d'utilisation de la sonde.

Selon une réalisation, le dispositif de conditionnement est déverrouillé une fois que le nom du patient a été identifié dans le système de traitement des données D, dans l'appareil d'imagerie ou dans l'appareil indépendant et portable. Une telle réalisation permet d'associer de façon simple les caractéristiques de désinfection de la sonde au patient avec lequel cette sonde est utilisée.

Le système d'imagerie avec lequel est utilisé la sonde peut être agencé pour ne pas fonctionner avec une sonde dont toutes les informations pertinentes n'ont pas été transmises par le dispositif de conditionnement 1.

Une fois que le déverrouillage du dispositif de conditionnement 1 a été commandé, le système de traitement des données D émet une information selon laquelle la sonde 3 n'est plus acceptable par un autre dispositif de conditionnement 1 et doit être à nouveau désinfectée, comme représenté par la flèche F₇ de la Fig. 2. Après utilisation, la sonde 3 est renvoyée à l'étape de désinfection A. Cette sonde ne sera à nouveau acceptable par un dispositif de conditionnement 1 qu'après cette étape 1.

Le dispositif de conditionnement 1 peut communiquer avec le(s) système(s) de traitement de données D à distance ou bien par une connexion filaire.

Un historique du suivi de l'utilisation de la sonde est également enregistré. De la sorte, après une utilisation de la sonde par un médecin, l'unité de traitement des données D peut élaborer des instructions de désinfection particulières (degré, type de désinfection) en fonction du type de traitement pour lequel la sonde a été utilisée.

Selon une réalisation, toutes les informations transmises au dispositif de conditionnement 1 sont également transmises à la sonde elle-même qui comprend des moyens de stockage de ces informations. Ainsi la sonde peut avoir les fonctionnalités décrites ci-dessus en ce qui concerne l'analyse de sa légitimité d'utilisation.

Ainsi, l'invention permet de garantir qu'une sonde particulière est toujours utilisée de façon légitime du point de vue de l'hygiène et d'éviter une utilisation dangereuse de cette sonde.

## Revendications

1. Dispositif de conditionnement (1) mobile d'un appareil médical (3), du type sonde ou similaire, ledit dispositif formant une enceinte (5) interne destinée à recevoir au moins une partie (2) de l'appareil médical (3), ladite enceinte pouvant être fermée hermétiquement, ledit dispositif comprenant des moyens de verrouillage (7) de la fermeture de l'enceinte (5), ledit dispositif étant **caractérisé en ce qu'**il comprend des moyens de réception (9) d'informations d'identification (Iᵢ) en provenance de l'appareil médical (2) et des moyens de réception d'informations de désinfection (I_{d}) et d'utilisation en provenance d'au moins un système de traitement d'informations (D), lesdits moyens de verrouillage (7) étant agencés pour se déclencher si les moyens de réception reçoivent une information selon laquelle l'appareil médical (3) identifié qu'il doit recevoir a été désinfecté et pour empêcher le verrouillage si les moyens de réception reçoivent une information selon laquelle l'appareil médical (3) n'a pas été désinfecté.

2. Dispositif de conditionnement selon la revendication 1, **caractérisé en ce qu'**il comprend des moyens d'émission d'informations relatives à l'appareil médical (3) qu'elle contient vers le système de traitement d'informations (D), lesdits moyens d'émission étant agencés pour émettre l'information d'identification (Iᵢ) de l'appareil médical (3) reçu dans l'enceinte (5) au système de traitement d'informations (D) lorsque les moyens de verrouillage (7) ont verrouillé la fermeture de l'enceinte (5).

3. Dispositif de conditionnement selon la revendication 1 ou 2, **caractérisé en ce que** les moyens de verrouillage (7) sont agencés pour déverrouiller la fermeture de l'enceinte (5) lorsque les moyens de réception (9) reçoivent une information selon laquelle l'appareil médical (3) va être utilisé du système de traitement d'informations (D).

4. Dispositif de conditionnement selon la revendication 3, **caractérisé en ce que** les moyens d'émission sont agencés pour informer le système de traitement d'informations (D) lorsque la fermeture de l'enceinte (5) est déverrouillée et que l'appareil médical (3) est sorti de l'enceinte.

5. Dispositif de conditionnement selon la revendication 4, **caractérisé en ce que** les moyens d'émission envoient les informations relatives à l'identification (Iᵢ) et à la désinfection (I_{d}) de l'appareil médical (3) que le dispositif (1) contient dans son enceinte (5) au système de traitement des informations (D) lorsque les moyens de verrouillage (7) déverrouille la fermeture de l'enceinte (5).

6. Dispositif de conditionnement selon l'une quelconque des revendications 1 à 5, **caractérisé en ce qu'**il comprend des moyens d'horodatage, les moyens de verrouillage (7) étant agencés pour déverrouiller la fermeture de l'enceinte (5) lorsque l'appareil médical (3) est présent dans l'enceinte (5) depuis un temps prédéterminé.

7. Dispositif de conditionnement selon l'une quelconque des revendications 2 à 6, **caractérisé en ce que** les moyens d'émission et de réception d'informations sont des moyens fonctionnant à distance.

8. Dispositif de conditionnement selon l'une quelconque des revendications 2 à 7, **caractérisé en ce que** les moyens d'émission et de réception d'informations comprennent des moyens de connexion filaires.

9. Dispositif de conditionnement selon l'une quelconque des revendications 1 à 8, **caractérisé en ce que** le dispositif de conditionnement (1) est autoclavable.

10. Système de suivi de l'utilisation d'un appareil médical de type sonde comprenant des moyens d'identification dudit appareil portés par l'appareil médical (3), au moins un système d'acquisition et de traitement d'informations (D) comprenant des moyens d'acquisition des informations d'identification (Iᵢ) dudit appareil, des moyens d'acquisition d'informations d'utilisation relatives à l'utilisation dudit appareil et d'informations de désinfection (I_{d}) relatives à la désinfection dudit appareil, des moyens d'horodatage et des moyens d'association des informations d'identification, d'utilisation, de désinfection dudit appareil et d'horodatage, ledit système étant **caractérisé en ce qu'**il comprend un dispositif de conditionnement (1) selon l'une quelconque des revendications 1 à 9, le dispositif de conditionnement (1) recevant l'appareil médical (3) entre les phases de désinfection et d'utilisation de l'appareil, le dispositif de conditionnement (1), l'appareil médical (3), le système d'acquisition et de traitement d'informations (D) étant aptes à communiquer entre eux afin de se transmettre des informations relatives à l'identification (Iᵢ) de l'appareil médical (3), à sa désinfection (I_{d}) et à son utilisation.

11. Système de suivi selon la revendication 10, **caractérisé en ce que** les appareils médicaux, du type enceinte de désinfection, enceinte de stockage, appareil d'imagerie, avec lesquels l'appareil médical (3) de type sonde doit fonctionner comprennent chacun des moyens d'acquisition des informations d'identification (Iᵢ), des moyens d'acquisition d'informations de désinfection (I_{d}) et d'informations d'utilisation et des moyens d'horodatage.

12. Système de suivi selon la revendication 10 ou 11, **caractérisé en ce que** les appareils médicaux, du type enceinte de désinfection, enceinte de stockage, appareil d'imagerie, avec lesquels l'appareil médical (3) de type sonde doit fonctionner comprennent chacun un système d'acquisition et de traitement d'informations (D).

13. Système de suivi selon la revendication 10, **caractérisé en ce que** les moyens d'acquisition des informations d'identification (Iᵢ), les moyens d'acquisition de désinfection (I_{d}) et d'informations d'utilisation, les moyens d'horodatage sont regroupé au sein d'un appareil indépendant et portable apte à communiquer avec un système de traitement d'informations (D).

## Claims

1. Movable conditioning apparatus (1) for a probe-type or similar medical device (3), said device forming an inner chamber (5) designed to receive at least a section (2) of the medical device (3), said chamber capable of being sealed, said device containing locking means (7) for the chamber closure (5), said device being **characterized by** the fact that it carries means for reception (9) of identification information (Iᵢ) from the medical device (2) and reception means for disinfection information (I_{d}) and usage information from at least one information processing system (D), said locking means (7) being arranged to engage if the reception means receive information indicating that the identified medical device (3) which it is to receive has been disinfected and to prevent locking if the reception means receive information indicating that the medical device (3) has not been disinfected.

2. Conditioning apparatus based on claim 1, **characterized in that** the means for transmission of information relating to the medical device (3) placed within it to the information processing system (D), said transmission means being arranged to issue information for identification (Iᵢ) of the medical device (3) received in the chamber (5) to the information processing system (D) whenever the locking means (7) have locked the chamber closure (5).

3. Conditioning apparatus based on claim 1 or 2, **characterized in that** the locking means (7) are arranged to unlock the chamber closure (5) whenever the reception means (9) receive information indicating that the medical device (3) will be used from the information processing system (D).

4. Conditioning apparatus based on claim 3, **characterized in that** the transmission means are arranged to notify the information processing system (D) whenever the chamber closure (5) is unlocked and the medical device (3) removed from the chamber.

5. Conditioning apparatus based on claim 4, **characterized in that** the transmission means send information relating to identification (Iᵢ) and disinfection (I_{d}) of the medical device (3) which the apparatus (1) contains in its chamber (5) to the information processing system (D) whenever the locking systems (7) unlock the chamber closure (5).

6. Conditioning apparatus based on any of claims 1 to 5, **characterized in that** it comprises time-stamping means and locking means (7) arranged to lock the chamber closure (5) whenever the medical device (3) is present in the chamber (5) for a predetermined period.

7. Conditioning apparatus based on any of claims 2 to 6, **characterized in that** the information transmission and reception means function remotely.

8. Conditioning apparatus based on any of claims 2 to 7, **characterized in that** the information-transmission and reception means comprise wired connection-means.

9. Conditioning apparatus based on any of claims 1 to 8, **characterized in that** the conditioning apparatus (1) is auto-clavable.

10. Usage monitoring system for probe-type medical device comprising means for identification of said device carried by the medical device (3), at least one information acquisition and processing system (D) comprising means for the acquisition of information (Iᵢ) about said device, means for the acquisition of information relating to usage of said device and disinfection information (I_{d}) relating to disinfection of said device, time-stamping means and means for combining information on identification, usage, disinfection of said device and time-stamping, said system being **characterized in that** it comprises a conditioning apparatus (1) based on any of claims 1 to 9, the conditioning apparatus receiving the medical device (3) between the device's disinfection and usage phases, the conditioning apparatus (1), the medical device (3), the information acquisition and processing system (D) being capable of communicating with each other in order to transmit between themselves information relating to identification (Iᵢ), the medical device (3), its disinfection (I_{d}) and use.

11. Monitoring system based on claim 10, **characterized in that** medical devices of the nature of the disinfection chamber, storage chamber and imaging apparatus with which the probe-type medical device (3) must operate each comprise identification information acquisition means (Iᵢ), and acquisition means for disinfection information (I_{d}) and usage and time-stamping information.

12. Monitoring system based on claim 10 or 11, **characterized in that** medical devices of the nature of the disinfection chamber, storage chamber and imaging apparatus, with which the probe-type medical device (3) must operate each comprise an information acquisition and processing system (D).

13. Monitoring system based on claim 10, **characterized in that** identification information acquisition means (Ii), disinfection information (I_{d}) and usage information means, and time-stamping means are combined in an independent portable device capable of communicating with an information processing system (D).

## Patentansprüche

1. Mobile Konditionierungsvorrichtung (1) für ein medizinisches Gerät (3) vom Typ Sonde oder Ähnlichem, bei der die betreffende Vorrichtung eine innere Kammer (5) bildet, die das medizinische Gerät (3) zumindest teilweise aufnehmen soll und die luftdicht verschlossen werden kann, bei der die betreffende Vorrichtung über Verriegelungsmittel (7) zum Verschluss der Kammer (5) verfügt, bei der die betreffende Vorrichtung **dadurch gekennzeichnet ist, dass** sie Mittel umfasst, mit denen vom medizinischen Gerät (2) kommende Identifizierungsinformationen (Iᵢ) sowie Informationen zur Desinfektion (I_{d}) und Benutzung, die von mindestens einem Informationsverarbeitungssystem (D) kommen, empfangen werden können, wobei die betreffenden Verriegelungsmittel (7) so eingerichtet sind, dass sie ausgelöst werden, wenn die Empfangsmittel eine Information erhalten, gemäß der das identifizierte medizinische Gerät (3), das aufgenommen werden soll, desinfiziert worden ist, und dass die Verriegelung verhindert wird, wenn die Empfangsmittel eine Information erhalten, gemäß der das medizinische Gerät (3) nicht desinfiziert worden ist

2. Konditionierungsvorrichtung gemäß dem Anspruch 1, bei der die betreffende Vorrichtung **dadurch gekennzeichnet ist, dass** sie über Mittel zum Senden von Informationen bezüglich des in ihr enthaltenen medizinischen Geräts (3) an ein Informationsverarbeitungssystem (D) verfügt, wobei die betreffenden Sendemittel so eingerichtet sind, dass sie die Identifizierungsinformation (Iᵢ) für das in der Kammer (5) enthaltene medizinische Gerät (3) an das Informationsverarbeitungssystem (D) senden, wenn die Verriegelungsmittel (7) den Verschluss der Kammer (5) verriegelt haben

3. Konditionierungsvorrichtung gemäß dem Anspruch 1 oder 2, bei der die betreffende Vorrichtung **dadurch gekennzeichnet ist, dass** die Verriegelungsmittel (7) so eingerichtet sind, dass sie den Verschluss der Kammer (5) entriegeln, wenn die Empfangsmittel (9) eine Information erhalten, gemäß der das medizinische Gerät (3) vom Informationsverarbeitungssystem (D) benutzt wird

4. Konditionierungsvorrichtung gemäß dem Anspruch 3, bei der die betreffende Vorrichtung **dadurch gekennzeichnet ist, dass** die Sendemittel so eingerichtet sind, dass sie das Informationsverarbeitungssystem (D) informieren, wenn der Verschluss der Kammer (5) entriegelt und das medizinische Gerät (3) aus der Kammer herausgenommen wird

5. Konditionierungsvorrichtung gemäß dem Anspruch 4, bei der die betreffende Vorrichtung **dadurch gekennzeichnet ist, dass** die Sendemittel die Informationen zur Identifizierung (Iᵢ) und Desinfektion (I_{d}) des medizinischen Geräts (3), das die Vorrichtung (1) in ihrer Kammer (5) enthält, an das Informationsverarbeitungssystem (D) schicken, wenn die Verriegelungsmittel (7) den Verschluss der Kammer (5) entriegeln

6. Konditionierungsvorrichtung gemäß einem beliebigen der Ansprüche 1 bis 5, bei der die betreffende Vorrichtung **dadurch gekennzeichnet ist, dass** sie über Mittel zur automatischen Aufzeichnung von Datum und Uhrzeit verfügt, wobei die Verriegelungsmittel (7) so eingerichtet sind, dass sie den Verschluss der Kammer (5) entriegeln, wenn sich das medizinische Gerät seit einer vorher festgelegten Zeit in der Kammer (5) befindet

7. Konditionierungsvorrichtung gemäß einem beliebigen der Ansprüche 2 bis 6, bei der die betreffende Vorrichtung **dadurch gekennzeichnet ist, dass** die Mittel zum Senden und Empfangen von Informationen Mittel sind, die mit Fernbetätigung funktionieren

8. Konditionierungsvorrichtung gemäß einem beliebigen der Ansprüche 2 bis 7, bei der die betreffende Vorrichtung **dadurch gekennzeichnet ist, dass** die Mittel zum Senden und Empfangen von Informationen drahtgebundene Anschlussmittel enthalten

9. Konditionierungsvorrichtung gemäß einem beliebigen der Ansprüche 1 bis 8, bei der die betreffende Vorrichtung **dadurch gekennzeichnet ist, dass** die Konditionierungsvorrichtung (1) als Autoklav benutzt werden kann

10. Überwachungssystem für die Benutzung eines medizinischen Geräts vom Typ Sonde, welches über Mittel zur Identifizierung des betreffenden Geräts mit dem medizinischen Gerät (3) als Informationsträger verfügt, zumindest über ein System zur Erfassung und Verarbeitung von Informationen (D), bestehend aus Mitteln zur Erfassung der Informationen für die Identifizierung (Iᵢ) des betreffenden Geräts, Mitteln zur Erfassung von Benutzungsinformationen in Bezug auf die Benutzung des betreffenden Geräts, Mitteln zur Erfassung von Desinfektionsinformationen (I_{d}) in Bezug auf die Desinfektion des betreffenden Geräts, Mitteln zur automatischen Aufzeichnung von Datum und Uhrzeit sowie Mitteln für die Verknüpfung der Informationen zur Identifizierung, Benutzung und Desinfektion des betreffenden Geräts und zur automatischen Aufzeichnung von Datum und Uhrzeit, wobei das betreffende System **dadurch gekennzeichnet ist, dass** es über eine Konditionierungsvorrichtung (1) gemäß einem beliebigen der Ansprüche 1 bis 9 verfügt und die Konditionierungsvorrichtung (1) das medizinische Gerät (3) zwischen den Phasen Desinfektion und Benutzung des Geräts aufnimmt, und wobei die Konditionierungsvorrichtung (1), das medizinische Gerät (3) und das System zur Erfassung und Verarbeitung von Informationen (D) in der Lage sind, miteinander zu kommunizieren, um Informationen in Bezug auf die Identifizierung (Iᵢ) des medizinischen Geräts (3), auf dessen Desinfektion (I_{d}) und dessen Benutzung zu übertragen

11. Überwachungssystem gemäß dem Anspruch 10, bei dem das betreffende System **dadurch gekennzeichnet ist, dass** medizinische Geräte wie Desinfektionskammern, Aufbewahrungskammern oder Geräte für bildgebende medizinische Verfahren, mit denen das medizinische Gerät (3) vom Typ Sonde zusammenarbeiten soll, jeweils über Mittel zur Erfassung der Identifizierungsinformationen (Iᵢ), Mittel zur Erfassung von Informationen bezüglich Desinfektion (I_{d}) und Benutzung sowie Mittel zur automatischen Aufzeichnung von Datum und Uhrzeit verfügen

12. Überwachungssystem gemäß dem Anspruch 10 oder 11, bei dem das betreffende System **dadurch gekennzeichnet ist, dass** medizinische Geräte wie Desinfektionskammern, Aufbewahrungskammern oder Geräte für bildgebende medizinische Verfahren, mit denen das medizinische Gerät (3) vom Typ Sonde zusammenarbeiten soll, jeweils über ein System zur Erfassung und Verarbeitung von Informationen (D) verfügen

13. Überwachungssystem gemäß dem Anspruch 10, bei dem das betreffende System **dadurch gekennzeichnet ist, dass** die Mittel zur Erfassung der Identifizierungsinformationen (Iᵢ), die Mittel zur Erfassung von Informationen bezüglich Desinfektion (I_{d}) und Benutzung sowie die Mittel zur automatischen Aufzeichnung von Datum und Uhrzeit in einem unabhängigen, tragbaren Gerät, das in der Lage ist, mit einem Informationsverarbeitungssystem (D) zu kommunizieren, zusammengefasst sind
